# EUROPEAN PATENT APPLICATION

(11) **EP 1 964 559 A1**
(43) Date of publication of application: **03.09.2008**
(21) Application number: 08101849.1
(22) Date of filing: 21.02.2008
(51) Int. Cl.: A61K 31/385, A61K 31/685, A61K 36/16, A61K 9/127, A61P 25/28

(54) **Compositions for the prevention and treatment of vascular dementia**

(30) Priority: 28.02.2007 IT MI20070394
(71) Applicant: Pharmaval S.r.L., 36050 Vicenze (IT)
(72) Inventor: Rachela, Enrico, 36050, Bolzano Vicentino (VI) (IT); D'Andrea, Giovanni, 36050, Bolzano Vicentino (VI) (IT)
(74) Representative: Minoja, Fabrizio

(57) **Abstract**

This invention relates to compositions for the prevention and treatment of vascular dementia containing pentacyclic diterpenes and dimeric flavones obtained from Ginkgo biloba, and thioctic acid.

## Description

This invention relates to compositions for the prevention and treatment of vascular dementia, containing pentacyclic diterpenes and dimeric flavones obtained from Ginkgo biloba, and thioctic acid.

### Introduction

Vascular dementia is a form of dementia secondary to a cerebral deficit or damage with a vascular basis, which involves an insufficient supply of energy, namely oxygen and glucose. Vascular dementia, unlike other forms of dementia, can therefore be treated by treating the underlying vascular disorder.

Ginkgo biloba extract is chemically characterised by the presence of two different active fractions: ginkgo flavone glycosides (24%) and terpenoids (6%), which include pentacyclic diterpenes. The second of these fractions is a powerful anti-PAF agent. Platelet-Activating Factor (PAF), which is definitely involved in the vascular disorder, is released by proinflammatory cells and cells of various other types due to the action of some acetyltransferases. Molecules which effectively counteract the action of that substance are the pentacyclic diterpenes (ginkgolides A, B, C, J and M) present in the terpene fraction obtained from Ginkgo biloba.

Numerous pharmacological studies have demonstrated that the mixture of these diterpenes is a true PAF-antagonist; for example, it prevents bronchoconstriction and/or reduces it by 90% in guinea pigs immunised against ovoalbumin or suffering from histamine-inoculum induced shock.

From the clinical standpoint ginkgolide B, the most active of the diterpenes, has proved effective in the treatment of asthma, as demonstrated by studies conducted on allergic and/or asthmatic patients during bronchoconstriction attacks. Finally, the mixture of these diterpenes prevents the release of histamine into proinflammatory cells derived from asthma patients.

Using a specific extraction process it is possible to obtain, from the leaves of Ginkgo biloba, another particular molecular fraction not contained in the conventional standardised Ginkgo biloba extract. This fraction corresponds to a highly purified mixture of five non-glycosylated biflavonic structures: amentoflavone, bilobetin, ginkgetin, isoginkgetin and sciadopitysin.

As demonstrated by recent studies, these biflavones present a series of activities which make them essential in a product designed to counteract various inflammatory phenomena, and are powerful extenders of the half-life of nitric oxide.

Thioctic acid, also known as alphalipoic acid, is a molecule well characterised for its antioxidant and endothelium-protecting potential. It is used in neurology, cardiology and diabetes.

### Description of the invention

It has now been discovered that compositions containing pentacyclic diterpenes obtained from Ginkgo biloba, dimeric flavones obtained from Ginkgo biloba, and thioctic acid are highly effective in the prevention and treatment of vascular dementia.

This invention therefore relates to compositions containing the following basic constituents:
a) pentacyclic diterpenes obtained from Ginkgo biloba,
b) dimeric flavones obtained from Ginkgo biloba, and
c) thioctic acid
for the prevention and treatment of vascular dementia.

In accordance with a preferred aspect of the invention, the pentacyclic diterpenes and dimeric flavones obtained from Ginkgo biloba will be present in the compositions in complexed form with phospholipids, as marketed under the brand name Phytosome®.

The basic constituents of the invention will be present in the compositions in the following weight ranges:
a) pentacyclic diterpenes obtained from Ginkgo biloba or the corresponding complex with phospholipids: 1-120 mg,
b) dimeric flavones obtained from Ginkgo biloba or the corresponding complex with phospholipids: 1-120 mg, and
c) thioctic acid: 1-1500 mg.

According to a particularly preferred aspect, the compositions according to the invention will contain the following quantities of the following basic constituents:
a) pentacyclic diterpenes obtained from Ginkgo biloba phytosome: 60 mg,
b) dimeric flavones obtained from Ginkgo biloba phytosome: 30 mg, and
c) thioctic acid: 100 mg.

It has been found that the compositions according to the invention exert a marked endothelial protective action, characterised by a high antiradical and PAF-antagonist power that prevents or slows the progress of the illness, which normally leads to states of full-blown vascular dementia.

This activity is even more evident when the two derivatives of G. biloba are used in the form complexed with phospholipids, in particular with soya distearoylphosphatidylcholine.

The action of the compositions according to the invention can be partly explained as follows.

Vascular dementia is caused by ischaemia of the frontal subcortical regions. This ischaemia is caused by thrombosis of the terminal vessels of the medullary arterioles perforating from the cerebral convexity. The process is due to exhaustion of the endothelial function characterised by production of nitric oxide (NO, a vasodilator and platelet deactivator) and an increase in the release of endothelin and platelet-activating factor (PAF) by the circulating cells (white blood cells and platelets). This homeostatic abnormality of the microcirculation leads to hypertension, progressive vasoconstriction, vascular degeneration and finally, thrombotic occlusion and cerebral atrophy. Moreover, PAF performs a proapoptotic and inflammatory action on the neurones per se, thus contributing to the progress of the primary dementia-inducing processes (Alzheimer's disease). The ginkgolides and other constituents of Ginkgo biloba have a protective effect on the microcirculation, inhibiting the action of PAF on the platelets and promoting vasodilatation, with an increase in nitric oxide. They also slow the neuronal apoptotic process with the same mechanism.

However, the effect obtained with the compositions according to the invention is far greater than that sum of the separate effects obtained by separate administration of the individual components of the association. This greater effect can be explained by a synergic mechanism at physiological level between the three constituents of the compositions in question.

The compositions according to the invention could be formulated suitably for oral administration, and prepared according to conventional methods well known in pharmaceutical technology, such as those described in Remington's Pharmaceutical Handbook, Mack Publishing Co., N.Y., USA, using excipients, diluents, fillers and anti-caking agents acceptable for their final use.

A preferred aspect of the invention is constituted by solid oral differentiated-release forms, especially a slow-release bonded to a normal release layer.

Additional constituents able to strengthen the PAF-antagonist, NO-protector or merely antioxidant action of the formula can be used instead of (or in addition to) the three ingredients described so far. Polyphenol fractions obtained by extraction from plants (catechin, leucoanthocyanin, anthocyanoside and flavonolignan fractions), glutathione, coenzyme Q10 and SH (thiol) group providers should be considered In particular.

Some examples of formulations are set out below.

### Examples of formulations

1 ) TABLETS

| | |
|---|---|
| Ginkgo biloba terpenes phytosome | 60.000 |
| Ginkgo biloba biflavones phytosome | 30.000 |
| Thioctic acid | 100.000 |
| Dicalcium phosphate Di-Cafos C92-14 | 100.000 |
| Levilite | 5.000 |
| Edible gelatin | 15.000 |
| Dicalcium phosphate Di-Cafos C92-14 | 150.000 |
| Microcel 102/Endurance VE 090 | 149.000 |
| Explocel | 14.000 |
| Vegetable magnesium stearate | 5.000 |
| Aerosil 200 | 12.000 |

2) CAPSULES

| | |
|---|---|
| Ginkgo biloba terpenes phytosome | 30.000 |
| Ginkgo biloba biflavones phytosome | 15.000 |
| Thioctic acid | 100.000 |
| Dicalcium phosphate Di-Cafos C92-14 | 100.000 |
| Levilite | 5.000 |
| Microcel 102/Endurance VE 090 | 149.000 |
| Explocel | 14.000 |
| Aerosil 200 | 120.000 |

3) TWO-LAYER DIFFERENTIATED-RELEASE TABLETS

| 1st layer: slow release | |
|---|---|
| Dicalcium phosphate | 90 mg |
| Ginkgo biloba terpenes phytosome | 120 mg |
| Microcrystalline cellulose | 60 mg |

| 2nd layer: normal release | |
|---|---|
| Thioctic acid | 50 mg |
| Biflavone fraction | 30 mg |
| Vegetable magnesium stearate | 5 mg |
| Silicon dioxide | 5 mg |

4) SACHETS

| | |
|---|---|
| Ginkgo biloba phytosome | 60 mg |
| Biflavone fraction | 100 mg |
| Thioctic acid | 150 mg |
| Anhydrous citric acid | 590 mg |
| Aerosil 200 | 26 mg |
| Aspartame | 10 mg |
| Acesulfame K | 10 mg |

## Claims

1. Compositions containing pentacyclic diterpenes obtained from Ginkgo biloba, dimeric flavones obtained from Ginkgo biloba, and thioctic acid, together with conventional excipients and diluents.

2. Compositions as claimed in claim 1, containing pentacyclic diterpenes and dimeric flavones obtained from Ginkgo biloba in complexed form with phospholipids.

3. Compositions as claimed in claim 2, containing pentacyclic diterpenes and dimeric flavones obtained from Ginkgo biloba in complexed form with soya distearoylphosphatidylcholine.

4. Compositions as claimed in the preceding claims, wherein the active constituents are present in the following weight ranges:
a) pentacyclic diterpenes obtained from Ginkgo biloba or the corresponding complex with phospholipids: 1-120 mg,
b) dimeric flavones obtained from Ginkgo biloba or the corresponding complex with phospholipids: 1-120 mg, and
c) thioctic acid: 1-1500 mg.

5. Compositions as claimed in the preceding claims, wherein the active constituents are present in the following quantities:
a) pentacyclic diterpenes obtained from Ginkgo biloba phytosome: 60 mg,
b) dimeric flavones obtained from Ginkgo biloba phytosome: 30 mg, and
c) thioctic acid: 100 mg.

6. Compositions as claimed in the preceding claims, in the form of hard or soft gel capsules, sugar-coated pills, tablets, chewable tablets, effervescent tablets, granulates, powders, and controlled-release solid forms.

7. Use of a combination of:
a) pentacyclic diterpenes obtained from Ginkgo biloba,
b) dimeric flavones obtained from Ginkgo biloba, and
c) thioctic acid
to prepare a composition for the prevention and treatment of vascular dementia.

8. Use as claimed in claim 7, wherein the pentacyclic diterpenes and dimeric flavones obtained from Ginkgo biloba are in complexed form with phospholipids.
